# EUROPÄISCHE PATENTSCHRIFT

(11) **EP 0 601 313 B1**
(45) Veröffentlichungstag und Bekanntmachung des Hinweises auf die Patenterteilung: **03.03.1999**
(21) Anmeldenummer: 93116916.3
(22) Anmeldetag: 20.10.1993
(51) Int. Cl.: A61M 1/00, E05C 19/02, F04B 43/12

(54) **Einrichtung für mikrochirurgische Eingriffe am Auge eines Lebewesens**
Device for microsurgery on the eye of a living creature
Dispositif pour la microchirurgie de l'oeil d'un être vivant

(30) Priorität: 06.11.1992 CH 3448/92; 14.10.1993 CH 3096/93
(43) Veröffentlichungstag der Anmeldung: 15.06.1994
(60) Teilanmeldung: 98115138.4
(73) Patentinhaber: GRIESHABER & CO. AG SCHAFFHAUSEN, CH-8203 Schaffhausen (CH)
(72) Erfinder: Grieshaber, Hans R., CH-8200 Schaffhausen (CH); Demmerle, Rudolf, CH-8200 Schaffhausen (CH); Vogel, Urs, CH-8200 Schaffhausen (CH)
(74) Vertreter: Althoff, Gerhard

(56) Entgegenhaltungen:
- EP-A- 0 293 081
- EP-A- 0 362 822
- WO-A-86/06964
- US-A- 3 977 624
- US-A- 4 697 902
- US-A- 4 904 168
- US-A- 4 963 131
- US-E- R E33 250

## Beschreibung

Die Erfindung bezieht sich auf eine Einrichtung zur Aufnahme eines mit einer Irrigationsleitung und einer Aspirationsleitung versehenen Einschubelements zur Durchführung mikrochirurgischer Eingriffe am Auge eines Lebewesens, mit zwei in einem Gehäuse jeweils gegen die Rückstellkraft eines Zuggliedes schwenkbar gelagerten Verriegelungshebeln sowie einem an einer Achse gelagerten und von einem Antriebsaggregat drehbar angetriebenen sowie mit mehreren Rollen versehenen Pumpenrad, wobei die einzelnen Elemente derart im Innenraum des Gehäuses angeordnet sind, dass bei eingeführtem und von den Verriegelungshebeln gehaltenem Einschubelement die Irrigationsleitung mit einem Unterbrecherorgan und die mit einem Filter in Verbindung stehende Aspirationsleitung mit den Rollen des Pumpenrades peristaltisch zusammenwirkend verbunden sind.

Aus der US-A 4,904,168 ist eine zur Aufnahme eines kassettenförmigen und mit einer Irrigations- und einer Aspirationsleitung versehenen Einschubelements ausgebildete Vorrichtung bekannt, welche ein durch einen Deckel verschliessbares Gehäuse sowie im Innenraum desselben zwei im Abstand zueinander angeordnete und jeweils um einen Achszapfen gegen die Rückstellkraft einer Feder schwenkbare Verriegelungshebel sowie ein von einem Elektromotor um eine Achse rotierend antreibbares Pumpenrad umfasst, wobei das Pumpenrad mit daran verteilt angeordneten Rollen in der Funktionsstellung mit der zugeordneten Aspirationsleitung peristaltisch zusammenwirkend in Eingriff bringbar ist.

Eine weitere Einrichtung zur Aufnahme eines kassettenförmigen sowie mit einer Irrigations- und einer Aspirationsleitung versehenen Einschubelements ist aus der EP-A 0 362 822 bekannt, wobei die Einrichtung ein mit einer schwenkbar gelagerten Frontplatte versehenes Gehäuse, ein darin angeordnetes und elektromotorisch um eine Achse rotierend angetriebenes Pumpenrad sowie das damit in Eingriff bringbare und kassettenförmig ausgebildete Einschubelement umfasst, wobei in der Funktionsstellung das Pumpenrad mit daran angeordneten Rollen mit der am Einschubelement angeordnete Aspirationsleitung peristaltisch zusammenwirkend in Eingriff steht und das Einschubelement durch entsprechend ausgebildete Verschlusselemente am Gehäuse gehalten ist.

Weitere Einrichtungen mit mindestens einem Pumpenrad sowie einem damit in Eingriff bringbaren Einschubelement in Form eines kassettenförmigen Schlauchträgers sind aus den Druckschriften (WO 86/06964; EP-A 0 293 081; US-A 4,963,131) bekannt.

Der Erfindung liegt die Aufgabe zugrunde, eine Einrichtung gemäss dem Oberbegriff des Patentanspruchs 1 dahingehend zu verbessern und so auszubilden, dass beim Einführen des Einschubelements ein exaktes und betriebssicheres Zusammenwirken der daran angeordneten Funktionselemente mit den einzelnen, im Innenraum des Gehäuses angeordneten Funktionselementen erreicht wird.

Die Erfindung löst die Aufgabe dadurch, dass der als Kammer ausgebildete Innenraum für das in das Gehäuse einführbare Einschubelement durch mehrere in Einführrichtung orientierte und quer dazu in parallelem Abstand zueinander angeordnete Zwischenwände in einzelne Funktionsebenen unterteilt ist, wobei die erste Funktionsebene das an der ersten Zwischenwand angeordnete Antriebsaggregat, ein Belüftungsventil sowie das Unterbrecherorgan und die zweite Funktionsebene die beiden an der einen Seite der zweiten Zwischenwand schwenkbar gelagerten Verriegelungshebel und die dritte Funktionsebene das im Abstand zu der dritten Zwischenwand an der anderen Seite der zweiten Zwischenwand gelagerte Pumpenrad umfasst, welches über eine quer zu den Zwischenwänden orientierte und mit dem einen Ende in der dritten Zwischenwand gelagerte sowie mit dem anderen Ende mit dem Antriebsaggregat wirkverbundene Antriebswelle verbunden ist.

Weitere Merkmale der Erfindung ergeben sich aus der folgenden Beschreibung in Verbindung mit der Zeichnung und den Patentansprüchen.

Die Erfindung wird nachstehend anhand der Zeichnung beschrieben. Es zeigt:
**Fig.1** eine in perspektivischer Ansicht dargestellte ophthalmologische Einrichtung mit einem herausgezogen dargestellten und als Schlauchträger ausgebildeten Einschubelement;
**Fig.2** ein in grösserem Massstab und im Schnitt dargestelltes Teilstück eines Trägerelements für das Einschubelement mit in einer ersten Funktionsebene angeordneten Funktionselementen;
**Fig.3** das in grösserem Massstab und im Schnitt dargestellte Teilstück des Trägerelements mit einer in einer zweiten Funktionsebene angeordneten Einzugsmechanik für das teilweise herausgezogen dargestellte Einschubelement;
**Fig.4** das Teilstück des Trägerelements gemäss Fig.3 mit der zweiten Funktionsebene mit dem von der Einzugsmechanik in das Trägerelement eingezogenen Einschubelement;
**Fig.5** das in grösserem Massstab und im Schnitt dargestellte Teilstück des Trägerelements mit einer in einer dritten Funktionsebene angeordneten sowie mit dem Einschubelement im Eingriff stehenden Pumpeneinheit; und
**Fig.6** das im Schnitt und in Draufsicht dargestellte Trägerelement mit den drei in parallelem Abstand zueinander angeordneten Funktionsebenen und den jeweils in den einzelnen Ebenen angeordneten Funktionselementen.

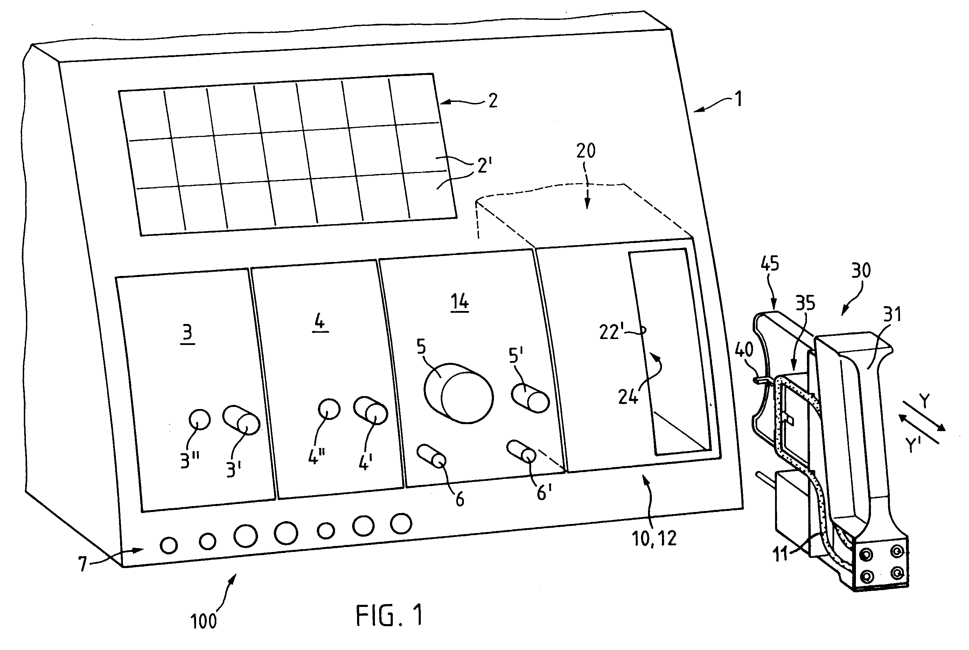

Fig.1 zeigt eine in perspektivischer Ansicht dargestellte und in der Gesamtheit mit 100 bezeichnete ophthalmologische Einrichtung zur Verwendung bei mikrochirurgischen Eingriffen am Auge eines Lebewesens. Die Einrichtung 100 umfasst mehrere, in einem Gehäuse 1 angeordnete und für den jeweiligen operativen Eingriff ausgebildete Funktionseinheiten 3, 4, 10, 12 und 14. Die einzelnen Funktionseinheiten 3, 4, 10, 12 und 14 sind vorzugsweise als nicht näher dargestellte, auswechselbare Einschübe ausgebildet und von der Frontseite in das Gehäuse 1 einschiebbar. Die beiden ersten Funktionseinheiten 3 und 4 sind mit nicht dargestellten Mitteln versehen, mittels welcher beim operativen Eingriff der Augeninnenraum beleuchtet werden kann. Hierzu wird ein nicht dargestelltes, an dem einen Ende mit einer Lichtquelle und an dem anderen Ende mit einem Anschlussteil versehenes Lichtleiterkabel an die entsprechend ausgebildeten Buchsen 3'' und/oder 4'' der Funktionseinheiten 3 und/oder 4 angeschlossen. Die für den operativen Eingriff erforderliche Lichtstärke kann über einen Drehknopf 3' und/oder 4' stufenlos eingestellt und reguliert werden. Weiterhin erkennt man in Fig.1 eine an der Stirnseite des Gehäuses 1 mit mehreren Anschlüssen versehene Steckerleiste 7, welche zum Anschluss zusätzlicher, nicht näher dargestellter Operationsinstrumente oder dergleichen vorgesehen ist.

An der Frontseite des Gehäuses 1 ist oberhalb der einzelnen Funktionseinheiten ein Bildfeld 2 angeordnet, welches beispielsweise in einzelne Anzeige- und Bedienungsfelder 2' mit LCD-Anzeige (Liquid Crystal Display) unterteilt ist. Das einzelne Anzeige- und Bedienungsfeld 2' dient als programmabhängig gesteuerter Informationsaustausch für den Benutzer der gesamten ophthalmologischen Einrichtung 100. Die einzelnen Anzeige- und Bedienungsfelder 2' sind durch geringfügigen Fingerdruck für die jeweilige Programmwahl betätigbar, wobei die angewählte Anzeige oder angewählten Anzeigen in bezug auf die nicht betätigten Anzeigen dann beleuchtet sind.

In der nachstehenden Beschreibung wird die erste Funktionseinheit 10 als Irrigationseinheit 10, die zweite Funktionseinheit 12 als Aspirationseinheit 12 bezeichnet. Die dritte Funktionseinheit 14 wird als Druckeinheit 14 bezeichnet. Die beiden Einheiten 10 und 12 sind über nicht dargestellte Versorgungs- und Entsorgungsleitungen miteinander verbunden und bilden zusammen ein nicht näher dargestelltes Aspirations- und Irrigationssystem.

Die Druckeinheit 14 hat mindestens einen Stutzen 6 zum Anschliessen einer Leitung sowie einen Einstellknopf 5 zum Regulieren des intraokularen Druckes (IOP) beim operativen Eingriff. Der Druckeinheit 14 kann eine nicht näher dargestellte Visko-Injektionseinrichtung zugeordnet werden, welche einen Stutzen 6' zum Anschliessen einer nicht dargestellten Leitung sowie einen Einstellknopf 5' zum Regulieren aufweist.

Die einzelnen, in Fig.1 nicht näher dargestellten Funktionselemente der Irrigationseinheit 10 sowie der Aspirationseinheit 12 stehen über ein in Fig.1 schematisch dargestelltes und in das Gehäuse 1 der Einrichtung 100 hineinschiebbares Einschubelement 30 miteinander in Wirkverbindung.

An dieser Stelle wird darauf hingewiesen, dass zur Aufnahme der einzelnen Funktionselemente und zur Aufnahme des Einschubelementes 30 in dem Gehäuse 1 eine entsprechend ausgebildete und mit einer Öffnung 22' versehene Kammer 24 angeordnet werden kann. Vorzugsweise ist jedoch ein in das Gehäuse 1 einsetzbares Trägerelement 20 vorgesehen, welches die Kammer 24 aufweist, in welche das Einschubelement 30 eingeführt und wieder herausgezogen werden kann.

Wie in Fig.1 schematisch dargestellt, kann das als Schlauchträger ausgebildete Einschubelement 30 gemäss Pfeilrichtung Y aus einem im Gehäuse 1 angeordneten, hier nicht näher dargestellten Trägerelement 20 herausgezogen und in Pfeilrichtung Y' wieder in die Kammer 24 des Trägerelements 20 hineingeschoben werden. An dieser Stelle wird darauf hingewiesen, dass das in den Figuren 1, 3, 4 und 5 dargestellte Einschubelement 30 mit den daran angeordneten Funktionselementen 11, 13, 31, 40, 45 und 56, 56' nicht Gegenstand dieser Erfindung ist und deshalb nicht näher beschrieben wird.

In dem Trägerelement 20 sind mehrere, in Fig.1 nicht dargestellte, Funktionselemente angeordnet, mit welchen das Einschubelement 30 in eingeschobenem Zustand in Wirkverbindung steht. Die in verschiedenen Funktionsebenen im Trägerelement 20 angeordneten Funktionselemente sowie eine entsprechend ausgebildete Einzugs- und Verriegelungsmechanik für das Einschubelement 30 werden nachstehend beschrieben.

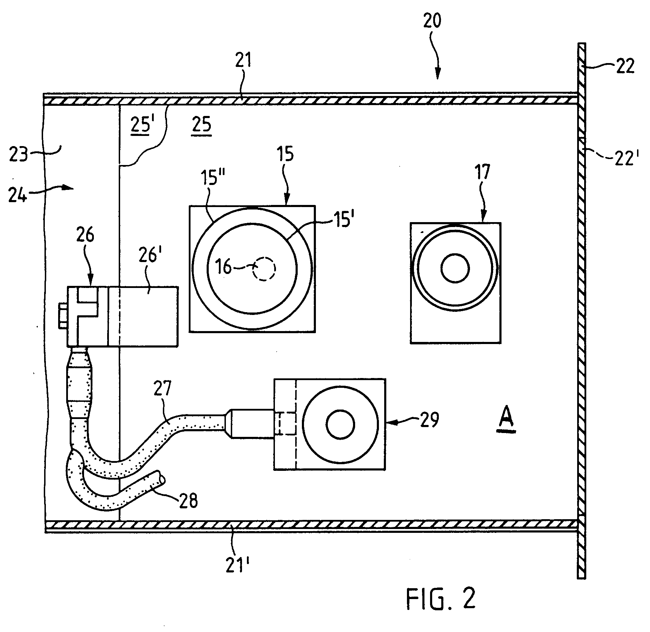

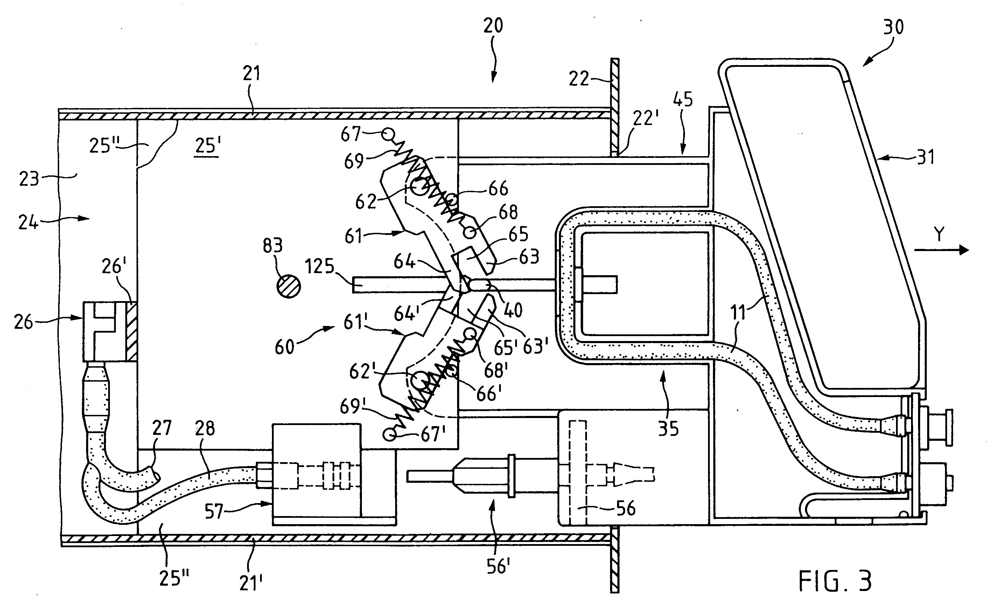

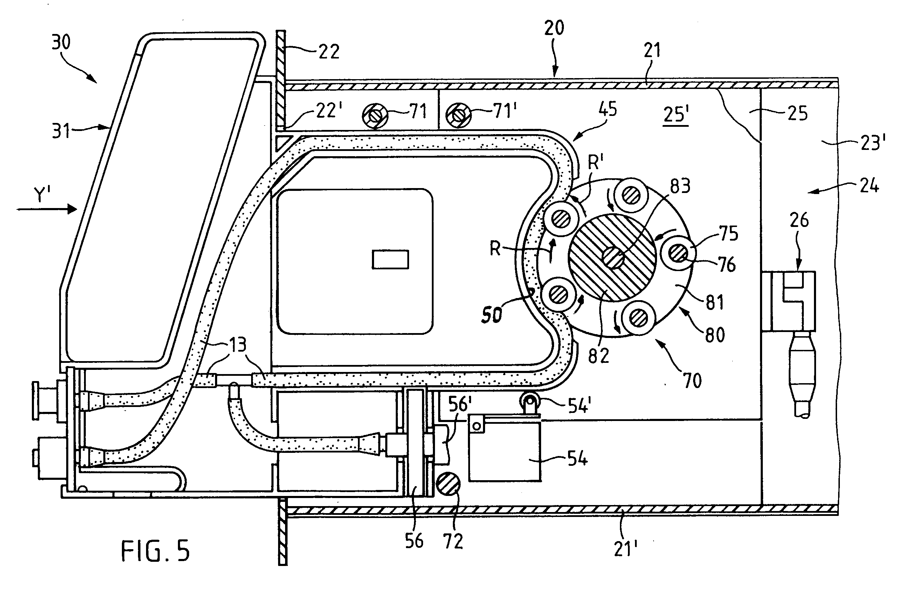

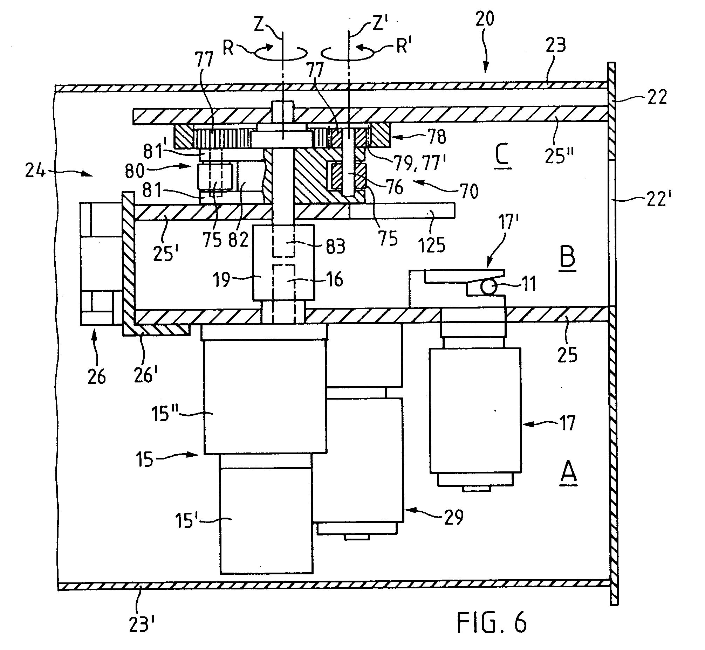

In Fig.2 ist ein in grösserem Massstab und im Schnitt dargestelltes Teilstück des Trägerelements 20 dargestellt. Das im wesentlichen kastenförmig ausgebildete Trägerelement 20 hat eine obere und eine untere Bodenplatte 21 und 21', zwei Seitenwände 23 und 23' (Fig.6), eine Stirnplatte 22 sowie eine nicht dargestellte Rückwand, wobei die Teile 21,21' und 23,23' sowie 22 im wesentlichen die zur Aufnahme der einzelnen Funktionselemente ausgebildete Kammer 24 bilden. In der Stirnplatte 22 ist eine Öffnung 22' (Fig.6) vorgesehen, durch welche das beispielsweise kassettenförmig ausgebildete Einschubelement 30 in die Kammer 24 einschiebbar ist. Zwischen den beiden Bodenplatten 21 und 21' ist, wie in Fig.2 dargestellt, eine erste Zwischenwand 25 angeordnet, welche mit nicht dargestellten Mitteln in dem Trägerelement 20 befestigt ist. An der ersten Zwischenwand 25 ist ein mit einem Getriebe 15'', einer Antriebswelle 16 (Fig.6) und mit einem Antriebsmotor 15' versehenes Antriebsaggregat 15 für eine Pumpeneinheit (Fig.5) angeordnet. Weiterhin ist an der ersten Zwischenwand 25 ein Magnetventil 17, ein Belüftungsventil 29 und ein über eine Leitung 27 damit in Verbindung stehendes Druckmessgerät 26 angeordnet. An die Leitung 27 ist ferner eine mit einem Anschlussgehäuse 57 (Fig.3) in Verbindung stehende Leitung 28 angeschlossen.

Die mit nicht dargestellten Mitteln an der ersten Zwischenwand 25 befestigten, schematisch dargestellten Funktionselemente 15, 17, 26 und 29 bilden zusammen mit der ersten Zwischenwand 25 im wesentlichen eine erste Funktionsebene A. Das Druckmessgerät 26 ist mit einer Halterung 26' an der ersten Zwischenwand 25 sowie an einer im Abstand dazu angeordneten zweiten Zwischenwand 25' (Fig.6) befestigt.

Fig.3 zeigt das in grösserem Massstab und im Schnitt dargestellte Trägerelement 20 sowie das in Pfeilrichtung Y teilweise aus der Kammer 24 herausgezogen dargestellte Einschubelement 30. Weiterhin erkennt man die zweite Zwischenwand 25', welche ebenfalls mit nicht dargestellten Mitteln in dem Trägerelement 20 befestigt ist. An der zweiten Zwischenwand 25' ist eine Einzugs- und Verriegelungsmechanik 60 angeordnet, welche in der in Fig.3 dargestellten Stellung im wesentlichen ausser Eingriff des am Einschubelement 30 angeordneten Rastnockens 40 steht. An der zweiten Zwischenwand 25' ist das Anschlussgehäuse 57 angeordnet und mit nicht dargestellten Mitteln befestigt. Das Anschlussgehäuse 57 ist auf der einen Seite über die Leitungen 28 und 27 mit dem Druckmessgerät 26 und an der anderen Seite zur Aufnahme eines Kupplungsstücks 56' ausgebildet. Das Kupplungsstück 56' ist ein Teilstück des am Einschubelement 30 angeordneten Filters 56. Das Druckmessgerät 26 ist mit der Halterung 26' an der ersten sowie an der zweiten Zwischenwand 25, 25' angeordnet und befestigt.

Die ebenfalls an der zweiten Zwischenwand 25' angeordnete Einzugs- und Verriegelungsmechanik 60 umfasst im wesentlichen, wie in Fig.3 dargestellt, zwei korrespondierend zueinander angeordnete und jeweils mit dem einen Ende über einen Zapfen 62 und 62' an der zweiten Zwischenwand 25' gelagerte Verriegelungshebel 61 und 61'. Die beiden Verriegelungshebel 61 und 61' sind gegen die Rückstellkraft damit in Wirkverbindung stehender Zugglieder 69 und 69' um die Achsen der beiden Zapfen 62 und 62' schwenkbar an der zweiten Zwischenwand 25' gelagert. An dem anderen Ende ist der einzelne Verriegelungshebel 61 und 61' jeweils durch eine Ausnehmung 65 und 65' sowie zwei im Abstand zueinander angeordnete Stege 63 und 64 beziehungsweise 63' und 64' zum Umgreifen des am Einschubelement 30 angeordneten Rastnockens 40 gabelförmig ausgebildet. Die beiden Zugglieder 69,69' sind mit dem einen Ende jeweils an einem an der zweiten Zwischenwand 25' angeordneten und befestigten Zapfen 67 und 67' gehalten und stehen mit dem anderen Ende über jeweils an den beiden Verriegelungshebeln 61, 61' angeordnete und befestigte Zapfen 68 und 68' in Wirkverbindung. Die beiden Zugglieder 69 und 69' sind beispielsweise als Schraubenfedern ausgebildet und an beiden Enden mit angeformten, die Zapfen 67, 67' und 68,68' umgreifenden Bogenteilen (nicht bezeichnet) versehen.

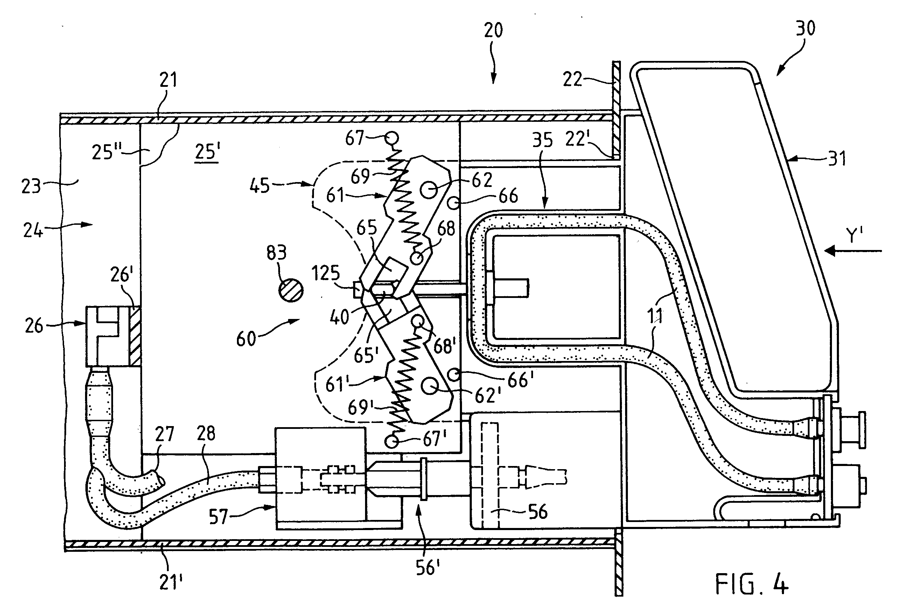

Beim Einführen des Einschubelements 30 in das Trägerelement 20 gemäss Pfeilrichtung Y' wird, wie in Fig.4 dargestellt, der am Einschubelement 30 angeformte Rastnocken 40 gegen die einen Gabelteile 64, 64' der beiden Verriegelungshebel 61,61' gedrückt. Hierbei werden die Verriegelungshebel 61,61' um die beiden Zapfen 62, 62' und dabei gleichzeitig die beiden Schraubenfedern 69, 69' um die Zapfen 67, 67' verschwenkt. Bei diesem Vorgang werden die beiden Schraubenfedern 69, 69' gespannt. Beim Erreichen und Überschreiten einer nicht dargestellten Totpunktstellung der beiden Verriegelungshebel 61, 61' wird durch die Rückstellkraft der in dieser Position gespannten Schraubenfedern 69, 69' das Einschubelement 30 schnappartig in die Kammer 24 des Trägerelements 20 gezogen. Bei diesem Bewegungsablauf wird das Einschubelement 30 mit der daran angeordneten zweiten Schlauchleitung 13 (Fig.5) mit einer in der Gesamtheit mit 70 bezeichneten Pumpeneinheit und das Kupplungsstück 56', wie in Fig.4 dargestellt, mit dem Anschlussgehäuse 57 in Eingriff gebracht.

Beim Herausziehen des Einschubelements 30 in Pfeilrichtung Y (Fig.3) drückt der Rastnocken 40 gegen die anderen Gabelteile 63,63' der beiden Verriegelungshebel 61,61', so dass diese mit den beiden Schraubenfedern 69,69' verschwenkt werden. Die Schwenkbewegung der beiden Verriegelungshebel 61,61' wird durch zwei entsprechend zugeordnete und an der zweiten Zwischenwand 25' befestigte Zapfen 66 und 66' begrenzt. In der Endstellung (Fig.3) der beiden Verriegelungshebel 61,61' ist der Rastnocken 40 des Einschubelements 30 ausser Eingriff der beiden Verriegelungshebel 61 und 61', so dass das Einschubelement 30 gemäss Pfeilrichtung Y aus der Kammer 24 des Trägerelements 20 beziehungsweise aus einer nicht dargestellten Kammer des Gehäuses 1 herausgezogen werden kann.

Beim Einführen des Einschubelements 30 in das Trägerelement 20 gemäss Pfeilrichtung Y' wird, wie in Fig.4 dargestellt, das Einschubelement 30 mit dem Rastnocken 40 vorzugsweise in einem in der zweiten Zwischenwand 25' angeordneten in Einschubrichtung orientierten Schlitz 125 einerseits in bezug auf die Einzugs- und Verriegelungsmechanik 60 und andererseits in bezug auf die Pumpeneinheit 70 geführt. Das Einschubelement 30 wird dabei mit dem mit der zweiten Schlauchleitung 13 versehenen zweiten Tragteil 45, wie in Fig.5 dargestellt, mit der an einer dritten Zwischenwand 25'' angeordneten Pumpeneinheit 70 exakt in Eingriff gebracht. An der dritten Zwischenwand 25'' können zur Führung des Einschubelements 30 weiterhin im Abstand zueinander angeordnete Rollen 71,71' sowie Anschläge 72 vorgesehen werden, durch welche ein Verkanten des Einschubelements 30 im Trägerelement 20 verhindert wird. An der dritten Zwischenwand 25'' ist weiterhin mit nicht dargestellten Mitteln ein mit einem Tastorgan 54' versehener Schalter 54 befestigt. Von dem mit einem Tastorgan 54' versehenen Schalter 54 wird für die Funktionsbereitschaft in der Endstellung ein Signal an die Pumpeneinheit 70 weitergegeben. Durch das Signal wird die Pumpeneinheit 70 betätigt.

Die über eine Antriebswelle 83 (Fig.6) und eine Kupplung 19 mit der Antriebswelle 16 des Antriebsaggregates 15 (Fig.2) in Wirkverbindung stehende Pumpeneinheit 70 umfasst, wie in Fig.5 dargestellt, ein in Pfeilrichtung R angetriebenes Pumpenrad 80. An dem Pumpenrad 80 sind in Umfangsrichtung im Abstand zueinander angeordnete Rollen 75 gelagert. Im dargestellten Ausführungsbeispiel sind an dem Pumpenrad 80 fünf am Umfang verteilt angeordnete Rollen 75 vorgesehen. Hierdurch wird erreicht, dass in jeder Stellung des Pumpenrades 80 mindestens zwei Rollen 75 mit der am zweiten Tragteil 45 angeordneten zweiten Schlauchleitung 13 (Fig.5) in Eingriff stehen. Die Rollen 75 sind jeweils um eine Achse 76 entgegen der Drehrichtung des Pumpenrades 80 in Pfeilrichtung R' angetrieben. Die entgegen der Pfeilrichtung R des Pumpenrades 80 in Pfeilrichtung R' angetriebenen Rollen 75 bewirken einen optimalen Funktionsablauf des peristaltisch arbeitenden Pumpenrades 80, ohne dass dabei auf die Schlauchleitung 13, welche an der kreisbogenförmigen Kurvenbahn 50 des Tragteils 45 anliegt, eine die Schlauchleitung 13 streckende Zugwirkung übertragen wird. Die an der dritten Zwischenwand 25'' angeordnete Pumpeneinheit 70 wird später in Verbindung mit Fig.6 noch im einzelnen beschrieben.

In Fig.6 ist das kastenförmig ausgebildete Trägerelement 20 mit den drei in parallelem Abstand zueinander angeordneten Funktionsebenen A, B und C im Schnitt und in Draufsicht dargestellt und man erkennt die beiden Seitenwände 23,23', die mit der Öffnung 22' versehene Stirnplatte 22 sowie die in der Kammer 24 angeordneten und mit nicht dargestellten Mitteln befestigten Zwischenwände 25,25' und 25''.

An der ersten Zwischenwand 25 sind mit nicht dargestellten Mitteln das mit dem Antriebsmotor 15' und dem Getriebe 15'' versehene Antriebsaggregat 15, das Belüftungsventil 29, das Druckmessgerät 26 sowie das Magnetventil 17 befestigt. Das Magnetventil 17 ist mit dem schematisch dargestellten Quetschelement 17' versehen, mittels welchem die elastische schlauchleitung 11 in eingeschobenem Zustand des Einschubelements 30 (Fig.4) je nach Ansteuerung des Magnetventils 17 abgeklemmt und wieder geöffnet werden kann. Die Schlauchleitung 11 wird zum Abklemmen mittels des Quetschelements 17' gegen einen nicht näher dargestellten und als Widerlager ausgebildeten Dorn des Quetschelements 17' gedrückt. Damit das Quetschelement 17' mit der Schlauchleitung 11 in Eingriff gebracht werden kann, sind in dem Einschubelement 30 entsprechend angeordnete Ausnehmungen vorgesehen. Das Druckmessgerät 26 ist mit der Halterung 26' an der ersten Zwischenwand 25 und der zweiten Zwischenwand 25' befestigt.

An der zweiten Zwischenwand 25' ist auf der der ersten Zwischenwand 25 zugewandten Seite die in Fig.3 und 4 dargestellte und vorstehend bereits beschriebene Einzugs- und Arretierungsmechanik 60 angeordnet.

An der der zweiten Zwischenwand 25' zugewandten Seite ist, wie in Fig.6 dargestellt, an der dritten Zwischenwand 25'' die in der Gesamtheit mit 70 bezeichnete Pumpeneinheit angeordnet und man erkennt das Pumpenrad 80, welches über eine Antriebswelle 83 und über die Kupplung 19 mit der Antriebswelle 16 des Antriebsaggregates 15 in Wirkverbindung steht. An der dritten Zwischenwand 25'' ist mit nicht dargestellten Mitteln ein mit einer Innenverzahnung 79 versehener Zahnkranz 78 befestigt. Mit der Innenverzahnung 79 des Zahnkranzes 78 stehen mehrere am inneren Umfang verteilt angeordnete und mit einer Aussenverzahnung 77' versehene Ritzel 77 in Eingriff, wobei jedes einzelne Ritzel 77 über eine Achse 76 mit dem Pumpenrad 80 in Wirkverbindung steht.

Das Pumpenrad 80 hat zwei durch ein scheibenförmiges Zwischenstück 82 in parallelem Abstand zueinander angeordnete Scheiben 81 und 81'. Zwischen den beiden Scheiben 81 und 81' sind die Rollen 75 angeordnet und mit der Achse 76 wirkverbunden. Die einzelnen Teile 75 bis 80 der Pumpeneinheit 70 stehen derart miteinander in Wirkverbindung, dass bei einer um die Achse Z orientierten Drehbewegung des Pumpenrades 80 in Pfeilrichtung R die am Pumpenrad 80 über die jeweilige Achse 76 mit dem Ritzel 77 und Zahnkranz 78 in Verbindung stehenden Rollen 75 um die Achse Z' in entgegengesetzter Drehrichtung in Pfeilrichtung R' drehen.

## Patentansprüche

1. Einrichtung zur Aufnahme eines mit einer Irrigationsleitung (11) und einer Aspirationsleitung (13) versehenen Einschubelements (30) zur Durchführung mikrochirurgischer Eingriffe am Auge eines Lebewesens, mit zwei in einem Gehäuse jeweils gegen die Rückstellkraft eines Zuggliedes schwenkbar gelagerten Verriegelungshebeln (61,61') sowie einem an einer Achse gelagerten und von einem Antriebsaggregat drehbar angetriebenen sowie mit mehreren Rollen versehenen Pumpenrad (80), wobei die einzelnen Elemente derart im Innenraum des Gehäuses angeordnet sind, dass bei eingeführtem und von den Verriegelungshebeln gehaltenem Einschubelement die Irrigationsleitung mit einem Unterbrecherorgan (17) und die mit einem Filter in Verbindung stehende Aspirationsleitung mit den Rollen des Pumpenrades peristaltisch zusammenwirkend verbunden sind, **dadurch gekennzeichnet**, dass der als Kammer (24) ausgebildete Innenraum für das in das Gehäuse (1) einführbare Einschubelement (30) durch mehrere in Einführrichtung orientierte und quer dazu in Parallelem Abstand zueinander angeordnete Zwischenwände (25,25',25'') in einzelne Funktionsebenen (A,B,C) unterteilt ist, wobei die erste Funktionsebene (A) das an der ersten Zwischenwand (25) angeordnete Antriebsaggregat (15), ein Belüftungsventil (29) sowie das Unterbrecherorgan (17) und die zweite Funktionsebene (B) die beiden an der einen Seite der zweiten Zwischenwand (25') schwenkbar gelagerten Verriegelungshebel (61,61') und die dritte Funktionsebene (C) das im Abstand zu der dritten Zwischenwand (25'') an der anderen Seite der zweiten Zwischenwand (25') gelagerte Pumpenrad (80) umfasst, welches über eine quer zu den Zwischenwänden (25,25',25'') orientierte und mit dem einen Ende in der dritten Zwischenwand (25'') gelagerte sowie mit dem anderen Ende mit dem Antriebsaggregat (15) wirkverbundene Antriebswelle (83) verbunden ist.

2. Einrichtung nach Anspruch 1, **dadurch gekennzeichnet**, dass die beiden mit dem einen Ende an der zweiten Zwischenwand (25') jeweils um einen Achszapfen (62,62') schwenkbar gelagerten Verriegelungshebel (61,61') an dem anderen Ende zum Erfassen eines quer zur Einführrichtung an dem Einschubelement (30) angeordneten Rastnockens (40) mit einer gabelförmig ausgebildeten Ausnehmung (65,65') versehen sind.

3. Einrichtung nach Anspruch 2, **dadurch gekennzeichnet**, dass der eine Verriegelungshebel (61') im Bereich der Ausnehmung (65') in bezug auf den anderen Verriegelungshebel (61) derart abgesetzt ausgebildet ist, dass die beiden Verriegelungshebel (61,61') in der den Rastnocken (40) umgreifenden Endstellung übereinanderliegend zueinander angeordnet sind.

4. Einrichtung nach den Ansprüchen 1 bis 3, **dadurch gekennzeichnet**, dass die zweite Zwischenwand (25') zur Führung des Einschubelements (30) einen dem Rastnocken (40) entsprechend ausgebildeten und in Einführrichtung orientierten Längsschlitz (125) aufweist.

5. Einrichtung nach Anspruch 1, **dadurch gekennzeichnet**, dass an der dem Pumpenrad (80) zugewandten Seite der dritten Zwischenwand (25'') ein mit einer Innenverzahnung (79) versehener Zahnkranz (78) sowie mehrere in Umfangsrichtung verteilte und mit der Innenverzahnung (79) in Eingriff stehende Zahnritzel (77) angeordnet sind, welche jeweils über einen Achskörper (76) mit den einzelnen am Pumpenrad (80) in Umfangsrichtung verteilten Rollen (75) in Eingriff stehen.

6. Einrichtung nach den Ansprüchen 1 und 5, **dadurch gekennzeichnet**, dass das Pumpenrad (80) zwei durch ein zylindrisches Zwischenstück (82) beabstandete und parallel zueinander angeordnete Scheiben (81,81') aufweist, zwischen welchen die in Umfangsrichtung verteilten und mit dem Achskörper (76) um dessen Achse (Z') drehbar gelagerten Rollen (75) angeordnet sind.

7. Einrichtung nach den Ansprüchen 1 und 5, **dadurch gekennzeichnet**, dass das Pumpenrad (80) um eine erste Achse (Z) in eine erste Drehrichtung und die einzelnen über den Achskörper (76) mit dem Zahnritzel (77) wirkverbundenen Rollen (75) jeweils um eine zweite Achse (Z') in entgegengesetzter Richtung drehbar angetrieben sind.

8. Einrichtung nach Anspruch 6, **dadurch gekennzeichnet**, dass die jeweils angetriebenen Rollen (75) derart zwischen den beiden Scheiben (81,81') angeordnet und gelagert sind, dass ein theoretischer und durch die einzelnen in Umfangsrichtung verteilt zueinander angeordneten Rollen (75) gebildeter äusserer Abrollkreis grösser als der Aussendurchmesser der beiden Scheiben (81,81') des Pumpenrades (80) ausgebildet ist.

9. Einrichtung nach Anspruch 1, **dadurch gekennzeichnet**, dass an den Enden der beiden Zwischenwände (25,25') eine Halterung (26') für ein Druckmessgerät (26) angeordnet ist, welches über eine Leitung (27) mit dem an der ersten Zwischenwand (25) angeordneten Belüftungsventil (29) verbunden ist.

10. Einrichtung nach den Ansprüchen 1 und 9, **dadurch gekennzeichnet**, dass an der ersten Zwischenwand (25) ein Anschlussgehäuse (57) angeordnet ist, welches über eine Leitung (28) mit dem Druckmessgerät (26) sowie bei eingeführtem Einschubelement (30) mit dem daran angeordneten Filter (56) wirkverbunden ist.

11. Einrichtung nach den Ansprüchen 1 bis 10, **dadurch gekennzeichnet**, dass die einzelnen Funktionsebenen (A,B,C) mit den einzelnen an den Zwischenwänden (25,25',25'') angeordneten und befestigten Funktionselementen in einem kastenförmig ausgebildeten Trägerelement (20) angeordnet sind, welches auswechselbar in die Kammer (24) des Gehäuses (1) einschiebbar ausgebildet ist.

## Claims

1. Device for accommodating a plug-in element (30) provided with an irrigation conduit (11) and an aspiration conduit (13) which is used for carrying out microsurgery on the eye of a living creature, said device being provided with two latching levers (61, 61') that are respectively pivotally mounted against the restoring force of a tensioning member in a housing and also with a shaft mounted pump impeller (80) which is rotatably driven by a drive unit and is also provided with a plurality of rollers, wherein the individual elements are disposed in the interior of the housing in such manner that when the plug-in element is inserted and retained by the latching levers, the irrigation conduit is connected to an interrupter member (17) and the aspiration conduit communicating with a filter is connected to the rollers of the pump impeller for co-operating therewith in peristaltic manner, characterised in that the interior space in the form of a chamber (24)for the plug-in element (30) that is insertable into the housing (1) is subdivided into separate functional planes (A, B, C) by means of a plurality of parallel partition walls (25, 25', 25'') that are oriented in the direction of insertion while being mutually spaced in a direction perpendicular thereto, wherein the first functional plane (A) embraces the drive unit (15) which is disposed on the first partition wall (25), a ventilating valve (29) and also the interrupter member (17), and the second functional plane (B) embraces the two latching levers (61, 61') which are pivotally mounted on the one side of the second partition wall (25'), and the third functional plane (C) embraces the pump impeller (80) which is mounted on the other side of the second partition wall (25') and spaced from the third partition wall (25''), said pump impeller (80) being connected via a drive shaft (83) which is oriented perpendicularly to the partition walls (25, 25', 25'') and which is mounted at the one end in the third partition wall (25'') and is also actively linked to the drive unit (15) at the other end.

2. Device in accordance with Claim 1, characterised in that the two latching levers (61, 61'), which are pivotally mounted about a respective swivel pin (62, 62') on the second partition wall (25') at their one ends, are provided with a fork-like recess (65, 65') at their other ends for engaging a latching cam (40) which is arranged on the plug-in element (30) transversely of the direction of insertion.

3. Device in accordance with Claim 2, characterised in that, in the vicinity of the recess (65'), the one latching lever (61') is offset relative to the other latching lever (61) such that the two latching levers (61, 61') mutually overlap when in their end position in which they embrace the latching cam (40).

4. Device in accordance with Claims 1 to 3, characterised in that the second partition wall (25') comprises an elongated slit (125) which is constructed in correspondence with the latching cam (40) and is oriented in the direction of insertion for guiding the plug-in element (30).

5. Device in accordance with Claim 1, characterised in that, on the side of the third partition wall (25'') facing the pump impeller (80), there is disposed an internally toothed (79) gear ring (78) and also a plurality of circumferentially distributed pinions (77) which engage with the internal teeth (79) and also engage via a respective axle shaft (76) with the individual rollers (75) that are circumferentially distributed on the pump impeller (80).

6. Device in accordance with Claims 1 and 5, characterised in that the pump impeller (80) comprises two mutually parallel discs (81, 81') which are spaced apart by a cylindrical spacer (82) and between which are disposed the circumferentially distributed rollers (75) that are rotatably mounted with the axle shaft (76) about the axis (Z') thereof.

7. Device in accordance with Claims 1 and 5, characterised in that the pump impeller (80) is rotatably driven about a first axis (Z) in a first direction of rotation and the individual rollers (75) actively linked via the axle shaft (76) to the pinion (77) are each rotatably driven about a second axis (Z') in the opposite direction.

8. Device in accordance with Claim 6, characterised in that the respectively driven rollers (75) are disposed and mounted between the two discs (81, 81') in such manner that a theoretical outer rolling circle formed by the individual, mutually circumferentially distributed rollers (75) is formed which is greater than the outer diameter of the two discs (81, 81') of the pump impeller (80).

9. Device in accordance with Claim 1, characterised in that a mount (26') for a pressure measuring apparatus (26) is disposed at the ends of the two partition walls (25, 25'), said apparatus being connected via a conduit (27) to the ventilating valve (29) disposed on the first partition wall (25).

10. Device in accordance with Claims 1 and 9, characterised in that a terminal housing (57) is disposed on the first partition wall (25) and is actively linked via a conduit (28) to the pressure measuring apparatus (26) and also to the filter (56) disposed on the plug-in element (30) when this has been inserted.

11. Device in accordance with Claims 1 to 10, characterised in that the individual functional planes (A, B, C) together with the individual functional elements arranged on and attached to the partition walls (25, 25', 25'') are disposed in a box-like support element (20) which is constructed such as to be insertable in replaceable manner into the chamber (24) of the housing (1).

## Revendications

1. Dispositif pour la microchirurgie de l'oeil d'un être vivant, servant de logement pour un élément encastrable (30) équipé d'un conduit d'irrigation (11) et d'un conduit d'aspiration (13), avec deux leviers de verrouillage (61, 61') logés dans un boîtier et pivotant respectivement contre la force de rappel d'un élément de traction, ainsi qu'une roue de pompe rotative (80) pourvue de plusieurs galets, celle-ci étant montée sur un axe et actionnée par un équipement moteur et, les différents éléments étant disposés à l'intérieur du boîtier de sorte que lorsque l'élément encastrable est inséré et bloqué par les leviers de verrouillage, le conduit d'irrigation avec un organe interrupteur (17) et le conduit d'aspiration connecté à un filtre, sont concourants avec les galets de la roue de pompe, afin d'agir ensemble de façon péristaltique, caractérisé en ce que l'intérieur constituant une chambre (24) soit divisé, pour l'élément encastrable (30) qui est introduit dans le boîtier (1), en plusieurs niveaux de fonctionnement (A,B,C), à l'aide de plusieurs parois intermédiaires (25,25',25'') orientées dans la direction d'introduction et disposées transversalement à celle-ci à distance parallèle l'une par rapport à l'autre, le premier niveau de fonctionnement (A) comprenant l'équipement moteur (15) disposé à la première paroi intermédiaire (25), une vanne de ventilation (29) ainsi que l'organe interrupteur (17), le deuxième niveau de fonctionnement (B) comprenant les deux leviers de verrouillage (61,61') placés de façon pivotante sur une des faces de la deuxième paroi intermédiaire (25') et le troisième niveau de fonctionnement (C) comprenant la roue de pompe (80) placée à distance de la troisième paroi intermédiaire (25'') sur l'autre face de la deuxième paroi intermédiaire (25'), ladite roue de pompe étant reliée à l'arbre moteur (83) orienté transversalement par rapport aux parois intermédiaires (25,25',25'') et logé, à travers une extrémité dans la troisième paroi intermédiaire (25''), ledit arbre moteur étant en relation d'effet, à travers l'autre extrémité, avec l'équipement moteur (15).

2. Dispositif selon la revendication 1, caractérisé en ce que les deux leviers de verrouillage (61,61') placés de façon pivotante autour d'un tourillon (62,62') avec une extrémité, à la deuxième paroi intermédiaire (25'), sont pourvus à l'autre extrémité d'un creux (65,65') en forme de fourche, pour saisir une came à cran (40), disposée transversalement par rapport à la direction d'introduction, à l'élément encastrable (30).

3. Dispositif selon la revendication 2, caractérisé en ce que le creux (65') de l'un des leviers de verrouillage (61') est décalé par rapport à celui de l'autre lever de verrouillage (61) de sorte qu'en fin de course enveloppant la came à cran (40), les deux leviers de verrouillage (61,61') sont disposés de façon superposée.

4. Dispositif selon les revendications 1 à 3, caractérisé en ce que la deuxième paroi intermédiaire (25') présente, pour guider l'élément encastrable (30), une fente longitudinale (125) orientée dans la direction d'introduction et conçue conformément à la came à cran (40).

5. Dispositif selon la revendication 1, caractérisé en ce qu'une couronne (78) avec une denture intérieure (79) ainsi que plusieurs pignons (77), étant repartis en direction circonférentielle et en prise avec la denture intérieure (79) de la couronne, sont disposés à la troisième paroi intermédiaire (25'') sur le côté tourné vers la roue de pompe (80), lesdits pignons étant respectivement en prise, à travers un axe (76), avec les différents galets (75) repartis en direction circonférentielle à la roue de pompe (80).

6. Dispositif selon les revendications 1 et 5, caractérisé en ce que la roue de pompe (80) présente deux disques (81,81') disposés parallèlement et séparés par une pièce intermédiaire cylindrique (82), entre lesdits disques étant disposés et repartis en direction circonférentielle les galets (75) qui sont montés de façon mobile et rotative autour de l'axe (Z').

7. Dispositif selon les revendications 1 et 5, caractérisé en ce que la roue de pompe (80) est actionnée autour d'un premier axe (Z) dans une première direction de rotation et que les différents galets (75) étant en relation d'effet avec le pignon (77), à travers le corps d'essieu (76), sont actionnés autour d'un deuxième axe (Z') dans le sens opposé.

8. Dispositif selon la revendication 6, caractérisé en ce que les galets (75), respectivement actionnés, sont disposés et montés entre les deux disques (81,81') de sorte qu'un cercle de roulement extérieur théorique et formé par les différents galets (75), disposés et repartis en direction circonférentielle, soit plus grand que le diamètre extérieur des deux disques (81,81') de la roue de pompe (80).

9. Dispositif selon la revendication 1, caractérisé en ce qu'une fixation (26') pour un manomètre (26) est disposée aux extrémités des deux parois intermédiaires (25,25'), ledit manomètre étant relié, à travers un conduit (27), à la vanne de ventilation (29) disposée à la première paroi intermédiaire (25).

10. Dispositif selon les revendications 1 et 9, caractérisé en ce que sur la première paroi intermédiaire (25) est disposé un boîtier de raccordement (57) étant en relation d'effet, à travers un conduit (28), avec le manomètre (26), ainsi que lorsque l'élément encastrable (30) est inséré, avec le filtre disposé audit élément.

11. Dispositif selon les revendications 1 à 10, caractérisé en ce que les différents niveaux de fonctionnement (A,B,C) avec les différents éléments fonctionnels, disposés et fixés aux parois intermédiaires (25,25',25''), sont disposés dans un élément porteur (20) en forme de caisse, ledit élément étant constitué de façon échangeable et amovible dans la chambre (24) du boîtier (1).
